# EUROPEAN PATENT APPLICATION

(11) **EP 4 186 981 A1**
(43) Date of publication of application: **31.05.2023**
(21) Application number: 21210945.8
(22) Date of filing: 29.11.2021
(51) Int. Cl.: C12Q 1/6806

(54) **PARALLEL DIRECT ISOLATION AND MANIPULATION OF NUCLEIC ACID FROM CULTURED CELLS IN NANOLITER DROPLETS**

(71) Applicant: Karlsruher Institut für Technologie, 76131 Karlsruhe (DE)
(72) Inventor: LEVKIN, Pavel, 76344 Leopoldshafen (DE); CHAKRABORTY, Shraddha, 76351 Linkenheim-Hochstetten (DE); POPOVA, Anna, 76149 Karlsruhe (DE)
(74) Representative: Müller-Boré & Partner Patentanwälte PartG mbB

(57) **Abstract**

The present invention relates to methods for the direct and parallel isolation of nucleic acid from cells in a multitude of nano- or microdroplets, comprising steps of providing a patterned substrate comprising a solid support coated with or carrying a layer or film, said layer or film comprising a multitude of hydrophilic areas having a desired shape and size, surrounded by hydrophobic areas separating the hydrophilic areas into a desired spatial pattern; applying an aqueous fluid comprising cells to the multitude of hydrophilic areas, thereby forming an array of a multitude of separated aqueous fluid nano- or microdroplets, wherein the separated aqueous fluid nano- or microdroplets comprise cells; and isolating nucleic acid from the cells in the multitude of separated aqueous fluid nano- or microdroplets on the patterned substrate in parallel.

## Description

The present invention relates to methods for the direct and parallel isolation of nucleic acid from cells in a multitude of nano- or microdroplets, comprising steps of providing a patterned substrate comprising a solid support coated with or carrying a layer or film, said layer or film comprising a multitude of hydrophilic areas having a desired shape and size, surrounded by hydrophobic areas separating the hydrophilic areas into a desired spatial pattern; applying an aqueous fluid comprising cells to the multitude of hydrophilic areas, thereby forming an array of a multitude of separated aqueous fluid nano- or microdroplets, wherein the separated aqueous fluid nano- or microdroplets comprise cells; and isolating nucleic acid from the cells in the multitude of separated aqueous fluid nano- or microdroplets on the patterned substrate in parallel.

The isolation of nucleic acids, such as e.g. mRNA, from cells and the subsequent analysis, manipulation and/or processing of such isolated nucleic acids is a core element in a myriad of techniques and applications in biological, biochemical, biotechnological, pharmaceutical and/or medical research, as well as in a wide range of diagnostic and medical applications.

Respective techniques for the isolation of nucleic acids from cells known in the art are manifold. However, such conventional techniques usually require the sequential processing of one sample at a time in relatively large volumes, using e.g. conventional multi-well plates and/or tubes with a single sample per tube.

Thus, such conventional techniques are typically rather time- and labor-intensive, require larger amounts of reagents resulting in higher costs, are less sensitive and are prone to sample loss due to the use of large volumes, and thus require a higher number of cells.

Pertaining to the goal of simplifying the experimental course, some advancements have been made in developing protocols for parallel sample preparation based on multi-well plates, multiplexed solid-phase, droplet-based or nanowell-based microfluidic systems for direct isolation of nucleic acids from cell lysates. For example, an automated technique of mRNA extraction, purification, and reverse transcription, based on solid-phase microfluidics, has been reported. Further, a droplet microfluidics-based single step chemical lysis approach for high-throughput single cell reverse transcription polymerase chain reaction (RT-PCR) has been developed, wherein the cells are lysed in high pH buffer and merged with subsequent reagents for gene expression analysis. As one step further towards assessment of global transcriptomic landscape, a nanowell-based platform enabling parallel single cell lysis, barcoding of mRNA and transcriptomic analysis has been established. Furthermore, a protocol that involves encapsulation of cells within water-in-oil droplets for highly parallelized single cell transcriptomic studies has been reported.

However, also these more advanced techniques still suffer from various drawbacks and limitations. In particular, no platform/protocol has yet been reported that can support the entire workflow of cell culture, screening, phenotypic assessment, nucleic acid isolation and subsequent analysis and/or processing of the same. Further, working with microfluidics-based devices can be cumbersome, expensive, requires special experimental skills and increases the experimental costs due to overconsumption of reagents. Moreover, the shear force inside microfluidic channels might hinder the optimal yield of genetic material from cells. In addition, droplet microfluidics-based systems are not compatible with high-throughput addition of hundreds of different stimuli to each cell-containing droplet, and do not provide a surface to culture adherent cells for the incubation period required for most screening experiments. The need to use oil phase for droplet microfluidic raises the risk of cross-contamination between droplets in case cells are introduced to different small molecules.

Furthermore, microtiter plates and tubes are surrounded by boundaries that hinder efficient isolation of genetic material from low number of cells by preferential attachment of negatively charged nucleic acids to positively-charged polypropylene walls. Since microtiter plates are not compatible with thermocycler machines (due to the high temperatures needed), they cannot be used for the entire protocol, including both phenotypic outcome and gene expression analysis involving isolation of nucleic acids from cells. Further, the reaction volume in microtiter plates is limited to microliters, and cannot be miniaturized further.

With the recent advancements in the field of molecular genomics, it is crucial to develop novel protocols enabling cell culture and high-throughput screening along with direct evaluation of cellular phenotype and gene expression dynamics.

Accordingly, the technical problem underlying the present invention is the provision of methods for the isolation of nucleic acids from cells that provide superior time-, labor- and cost-efficiency, superior ease of use, a high sensitivity, the ability to work with a low number of cells, down to single cells, and enable an entire workflow of cell culture, screening, phenotypic assessment, nucleic acid isolation and subsequent analysis and/or processing, all in a high-throughput format.

The solution to the above technical problem is achieved by the embodiments characterized in the claims.

In particular, the present invention relates to a method for the direct and parallel isolation of nucleic acid from cells in a multitude of nano- or microdroplets, comprising the steps of:
(a) providing a patterned substrate comprising a solid support coated with or carrying a layer or film, said layer or film comprising
   (i) a multitude of hydrophilic areas having a desired shape and size, surrounded by
   (ii) hydrophobic areas separating the hydrophilic areas into a desired spatial pattern;
(b) applying an aqueous fluid comprising cells to the multitude of hydrophilic areas, thereby forming an array of a multitude of separated aqueous fluid nano- or microdroplets, wherein the separated aqueous fluid nano- or microdroplets comprise cells; and
(c) isolating nucleic acid from the cells in the multitude of separated aqueous fluid nano- or microdroplets on the patterned substrate in parallel.

As used herein, the term "direct isolation of nucleic acid from cells" indicates that according to the present invention, all steps from having intact living cells in the multitude of separated aqueous fluid nano- or microdroplets to having the isolated nucleic acid from said cells in the multitude of separated aqueous fluid nano- or microdroplets are performed in the individual nano- or microdroplets on the patterned substrate.

Further, as used herein, the term "parallel isolation of nucleic acid from cells" indicates that according to the present invention, nucleic acids are isolated from cells in the multitude of separated aqueous fluid nano- or microdroplets on the patterned substrate at the same time.

Nucleic acids that can be isolated in accordance with the present invention are not particularly limited and include any kind of nucleic acid the isolation of which from cells is of interest. Preferably, the nucleic acid is DNA, e.g. genomic DNA, or RNA, e.g. mRNA, miRNA or other types of RNA. In preferred embodiments, the nucleic acid is mRNA.

The patterned substrate used in the methods of the present invention is described in detail hereinafter. The hydrophilic areas that are surrounded by hydrophobic areas separating the hydrophilic areas into a desired spatial patterns can be referred to herein as "spots". Thus, in the following, the terms "area" and "spot" will be used synonymously. By way of example, the hydrophilic areas of the patterned substrates used in the present invention can be referred to as hydrophilic spots or just as spots.

Step (b) of the method of the present invention, *i.e.* the step of applying the aqueous fluid comprising the cells to a multitude of the hydrophilic areas, is a step wherein the aqueous fluid comprising the cells is applied to at least more than one, preferably to all spots. Alternatively, the aqueous fluid is applied to at least 5, 10, 15, 20, 30, 50, or 75% of all spots, more preferably to 100% of the spots. This can be effected by discontinuous wetting, wherein the aqueous fluid comprising the cells is applied to the whole patterned substrate or a part of said patterned substrate at the same time, or on a spot-by-spot basis using liquid dispensers.

According to the present invention, the aqueous fluid can be applied manually or with the help of an automated liquid-handling device. The aqueous fluid can be applied manually with the help of a pipette or with the help of an automated machine by spreading or rolling an amount of the aqueous fluid comprising the cells along the surface of the patterned substrate. The amount of fluid used in this respect is preferably an amount that is just sufficient to form nano- or microdroplets on all hydrophilic spots of the patterned substrate. The patterned substrate can also be immersed, manually or with the help of an automated machinery, into a solution or suspension of cells to form a Droplet Microarray. Using automated dispensers of any kind to spot substances or cells on individual hydrophilic spots, *i.e.,* on a spot-by-spot basis, is not excluded and can also be used if it is in favor of an application.

The number of cells present in an individual nano- or microdroplet can be chosen in dependence of the size of the hydrophilic spot and the intended specific application. Cell numbers per individual nano- or microdroplet can range from several thousand cells down to a single cell. Preferably, the number of cells per individual nano- or microdroplet is 1000 cells or less, more preferably 100 cells or less, more preferably 10 cells or less, and most preferably one single cell.

In specific embodiments, the methods of the present invention further comprise the step of (b-2) culturing the cells in the multitude of separated aqueous fluid nano- or microdroplets on the patterned substrate after step (b) and prior to step (c). Further, said methods can further comprise the step of (b-3) analyzing the cells in the multitude of separated aqueous fluid nano- or microdroplets on the patterned substrate by way of microscopy after step (b) and prior to step (c). In this context, step (b-3) can be performed one or multiple times, and in any order with step (b-2), *i.e*., prior to, during, and/or after step (b-2).

In specific embodiments, the nucleic acid to be isolated in the methods of the present invention is mRNA, and said methods further comprise the step of (d) synthesizing cDNA in the multitude of separated aqueous fluid nano- or microdroplets on the patterned substrate in parallel from the mRNA isolated in step (c).

In further specific embodiments, the methods of the present invention can further comprise one or more steps, selected from the group consisting of introducing barcodes to the nucleic acid, e.g. during the step of converting mRNA to cDNA, amplifying the nucleic acid, performing PCR (polymerase chain reaction) with the nucleic acid, performing qPCR (quantitative PCR) with the nucleic acid, and sequencing the nucleic acid, in the multitude of separated aqueous fluid nano- or microdroplets on the patterned substrate in parallel using the nucleic acid isolated in step (c).

Means for performing step (c) of the methods of the present invention, *i.e.,* isolating nucleic acid from the cells in the multitude of separated aqueous fluid nano- or microdroplets on the patterned substrate in parallel, are not particularly limited and are known in the art. The same applies for performing steps (b-2) and (b-3) as defined above, as well as any of the above additional steps.

Respective means include physical manipulation of the patterned substrate, e.g. shaking the same, incubating the patterned substrate in an incubator, incubating the patterned substrate on a heating plate or in a thermocycler, placing the patterned substrate in a magnetic field, and placing the patterned substrate in a centrifuge. Further, respective means include the addition of buffers (e.g. cell lysis buffers, hybridization buffers, wash buffers, nuclease-free water, and the like), reagents (e.g. magnetic beads, optionally labeled with e.g. nucleic acids or antibodies, nucleic acids such as primers, dNTPs, enzyme inhibitors, antibodies, and the like) or enzymes (e.g. reverse transcriptase, DNA polymerases, RNases, restriction enzymes, and the like).

All patterned substrate handling steps and all steps of adding something to the nano- or microdroplets can be performed manually or with the help of an automated machine, e.g. a liquid dispenser.

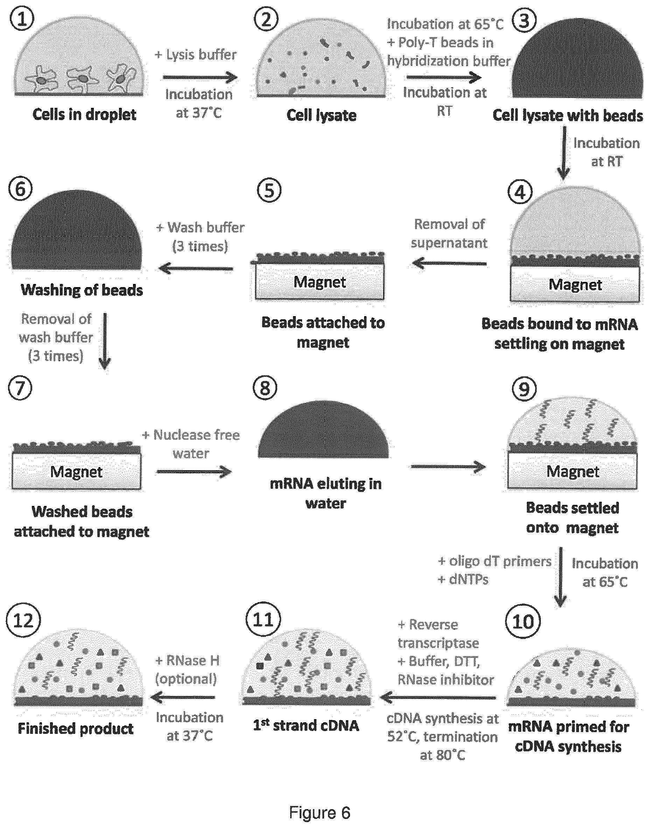

By way of example, Figure 6 shows a schematic overview of a method according to the present invention, wherein mRNA is isolated from cells and translated into cDNA. This method, performed in a multitude of nano- or microdroplets in parallel, encompasses the steps of (i) adding a cell lysis buffer to cells in a nano- or microdroplet, (ii) incubating the patterned substrate at 37°C to obtain a cell lysate, (iii) incubating the cell lysate with magnetic beads containing poly T-nucleic acid in a hybridization buffer, so that mRNA binds to said poly T-nucleic acid via their poly A-tail, (iv) placing the patterned substrate in a magnetic field, so that the magnetic beads attach to the magnet, (v) washing the magnetic beads, (vi) eluting the mRNA from the magnetic beads, (vii) adding oligo-dT primers and dNTPs to the mRNA, and (viii) adding reverse transcriptase and incubating the patterned substrate at a suitable temperature to allow the generation of cDNA.

The term "aqueous fluid" as used herein is not particularly limited and relates to any aqueous fluids that might be of interest for the formation of a respective nano-or microdroplet array. In particular, the aqueous fluid can be selected from the group consisting of water, aqueous solutions, and aqueous media.

According to the present invention, the aqueous fluid applied to the hydrophilic areas comprises cells. The term "cell" as used in this context is not particularly limited and encompasses any kind of prokaryotic and eukaryotic cells that might be of interest. Preferably, the cells are selected from at least one member of a group consisting of human, animal or plant primary cells, immortalized adherent and non-adherent cell lines, human and animal cancer cells, and human and animal stem cells.

The terms "nanodroplet" and "microdroplet" as used herein refer to the droplets formed on the patterned substrate in the methods of the present invention. Nano-or microdroplets can have a volume from about 5 nL to about 10 µL, preferably from about 50 nL to about 5 µL, from about 100 nL to about 5 µL, e.g. about 150 nL (for 1 mm diameter spots) or about 3 µL (for 3 mm diameter spots). The terms "nanodroplet" and "microdroplet" as used herein do not particularly limit the volume of the respective droplets and can be used interchangeably herein, wherein droplets with volumes in the nanoliter range are preferably designated as nanodroplets, and droplets with volumes in the microliter range are preferably designated as microdroplets.

The patterned substrate used in the present invention comprises a solid support, wherein the size and shape of said solid support is not particularly limited. Preferably, the solid support has a rectangular shape. The size of the solid support preferably is a standard size that allows the solid support to fit to laboratory equipment such as thermocyclers, heating plates, microscopes, and the like. Materials for the solid support are not particularly limited, but are preferably optically transparent materials such as suitable transparent plastic materials or glass. Preferably, the solid support is made of glass, wherein in a particularly preferred embodiment, the solid support is a standard microscope glass slide having dimensions of 2.5 cm × 7.5 cm.

The spatial pattern as a whole provided on the substrate can have any desired shape and size, and can fill the entire substrate or only one or more parts thereof. Thus, the term "spatial pattern" as used herein is not particularly limited and can encompass any desired pattern of nano- or microdroplets. Preferably, the pattern is chosen in a way that the density of nano- or microdroplets per area is maximized.

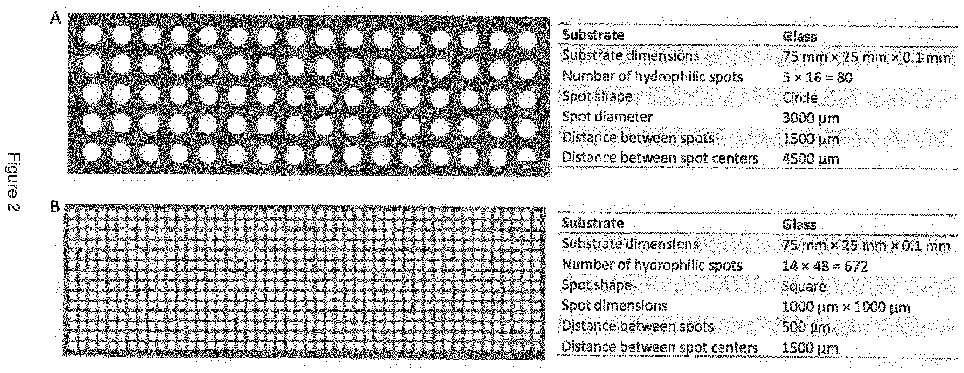

Hydrophilic spots can have any desired shape, e.g. a circular, square, or hexagonal shape, and can have also sharp-edged geometries such as triangular shapes. The size of the hydrophilic spots is not particularly limited and can be chosen in accordance with the desired cell number per nano- or microdroplet and the particular desired application. Typical sizes, e.g. for circular spots, range from spot diameters of about 100 µm to about 5 mm, preferably about 500 µm to 5 mm, e.g. about 1 mm or about 3 mm, with sizes for differently shaped spots resulting in comparable areas. The distance between spots can be chosen to maximize the density of nano- or microdroplets per areas on the one hand, while on the other hand assuring the prevention of cross-contamination from one nano-or microdroplet to the neighboring nano- or microdroplets. Typical distances between spots range from 100 µm to about 3 mm, preferably about 500 µm to 2 mm, e.g. about 500 µm or about 1.5 mm. The number of spots per patterned substrate is only limited by these parameters, *i.e.,* by the area of the patterned substrate, the size of the spots, and the required distance between spots. Thus, typical numbers of spots range from tens to hundreds or even several thousand of spots per patterned substrate. Exemplary values for all of the above parameters are indicated in Figure 2.

The nano- or microdroplets are preferably homogenous, *i.e.,* they all have the same size and shape, and they are separated, *i.e*., they are separated from each other by the hydrophobic areas separating the hydrophilic areas on which the nano- or microdroplets form. In this manner, the individual nano- or microdroplets are completely isolated. An array of nano- or microdroplets as formed in the method of the present invention is sometimes referred to as "Droplet Microarray" or "DMA" hereinafter.

The patterned substrate used in the present invention comprises a solid support that is coated with a layer or film, or carries a layer or film, said layer or film comprising the hydrophilic areas and hydrophobic areas forming the desired spatial pattern.

The patterned substrate used in the methods of the present invention is preferably a microarray, more preferably a cell microarray. Further, said patterned substrate preferably has a flat surface, and does not comprise any physical barriers, such as wells, recesses, indentations, depressions, ridges, or the like, separating the individual hydrophilic areas from each other.

In the following, the terms "hydrophilic" and "superwetting" will be used synonymously.

The combination of the watertight hydrophilic (superwettable) areas with highly efficient hydrophobic cell-barriers represents an approach for designing cell arrays and has the potential to push the spot-density towards its theoretical maximum that is only given by the necessary number of cells in each spot.

In general, surface properties can be classified into hydrophobic and hydrophilic surfaces depending on the value of the water contact angle (WCA). A surface having a WCA greater than 90° is referred to as hydrophobic, whereas a WCA smaller than 90° is referred to as hydrophilic. The maximum water contact angle on a smooth surface is about 120°. In practice, two types of WCA values are used: static and dynamic. Static water contact angles (qstat) are obtained by sessile drop measurements, where a drop is deposited on the surface and the value is obtained by a goniometer or a specialized software. Dynamic contact angles are non-equilibrium contact angles and are measured during the growth (advancing WCA qadv) and shrinkage (receding WCA qrec) of a water droplet. The difference between qadv and qrec is defined as contact angle hysteresis (CAH). In a preferred embodiment of the present invention, surfaces with high WCA, preferably greater than 130°, more preferably greater than 140°, most preferably greater than 150°, and/or low water CAH (less than about 30°, preferably less than about 20°, more preferably less than about 10°) are called superhydrophobic. Water droplets do not stick to such surfaces and simply roll off. In a preferred embodiment of the present invention, surfaces with both static, advancing and receding WCAs less than 20°, preferably less than 10°, more preferably less than 5°, most preferably close to or of 0° are called superhydrophilic. Water droplets are rapidly absorbed by such surfaces.

In a preferred embodiment of the present invention, the patterned substrate is a surface having hydrophilic, preferably superhydrophilic, patterns on a hydrophobic, preferably superhydrophobic, background.

The layer or film coated or carried on the patterned substrate is not particularly restricted as long as said layer or film can provide hydrophilic and hydrophobic regions. According to a preferred embodiment, the layer or film used in the present invention is either (i) a micro-to-nanorough inorganic film coated on the patterned substrate, e.g. using nanoparticles which are applied to the surface and then functionalized using hydrophilic and hydrophobic (fluorinated) groups to create patterns, (ii) a porous polymer layer or film coated on the patterned substrate, (iii) a micro-to-nanorough layer generated by e.g. laser structuring, or (iv) a smooth coating with omniphilic-omniphobic patterning, as known in the art. Preferably, these layers or films are biocompatible, i.e. they do not impair or negatively affect the growth, proliferation or well-being of the cells that are trapped in the nano- or microdroplets formed, and do not impair the structural or functional integrity of any biologically active compounds, such as nucleic acids, peptides, proteins, and enzymes, that may be present in the nano- or microdroplets.

Superhydrophobic and superhydrophilic materials are known in the art. Within the scope of the present invention, the layer can be a porous polymer layer, a micro-to-nanorough inorganic film, a rough hydrophobic-hydrophilic coating, a micro-to-nanorough layer, or a smooth omniphobic-omniphilic coating, as known in the art. Respective superhydrophobic-hydrophilic patterns can be produced by methods known in the art. For example, such superhydrophobic-hydrophilic patterns can be produced by making micro-nano rough coatings using nanoparticles, laser structuring, or other methods known in the art. In addition, smooth coatings with omniphilic-omniphobic patterning can be used.

According to the present invention, the thickness of the layer or film is not limited. Regarding transparency properties or reducing manufacturing costs, a thin layer or film is preferred. Particularly preferred is a layer or film having a thickness of 45 µm or less, preferably 30 µm or less, and more preferably 15 µm or less.

The very good wetting property of the surface of the layer or film inside hydrophilic areas guarantees an easy and homogeneous spreading of the solutions inside the areas independent of their geometry. In addition, the hydrophobic, preferably superhydrophobic, areas prevent cells from migrating between the adjacent areas, as well as cross-contamination between individual nano- or microdroplets.

The solid support may be of any material known in the art which is solid, preferably at a temperature within the range of -30°C to 130°C, more preferably within the range of 0°C to 40°C, more preferably at room temperature. Preferably, the solid support is optically transparent, wherein a glass solid support is particularly preferred. Such a glass solid support may be activated and/or modified prior to use.

As used herein, the term "comprising"/"comprises" expressly includes the terms "consisting essentially of"/"consists essentially of" and "consisting of"/"consists of", *i.e.,* all of said terms are interchangeable with each other herein.

Further, as used herein, the term "about" preferably represents a modifier of the specified value of ± 10%, more preferably ± 10%, ± 8%, ± 6%, ± 5%, ± 4%, ± 3%, ± 2%, ± 1%, or ± 0.5%. Thus, by way of example, the term "about 100" can include the ranges of 90 to 110, 92 to 108, 94 to 106, 95 to 105, 96 to 104, 97 to 103, 98 to 102, 99 to 101, or 99.5 to 100.5.

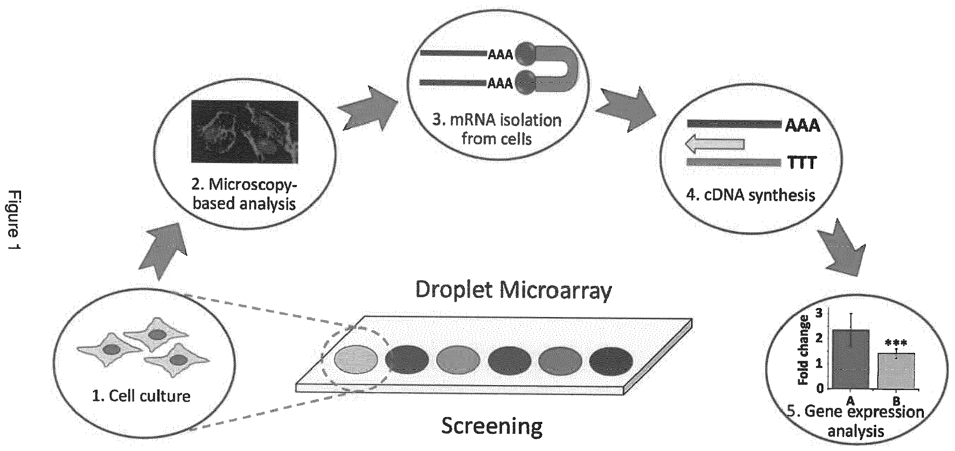

The present invention provides a novel 'Seq-on-a-Chip' methodology for the isolation of nucleic acids (e.g. mRNA or DNA) directly from cultured cells, down to a single cell per nano- or microdroplet, in parallel within hundreds of nanoliter droplets on a Droplet Microarray (DMA) platform, optionally followed by modification of the nucleic acids, e.g. mRNA to cDNA conversion, ligation of barcodes and amplification of nucleic acids, directly on the DMA chip in individual separated nanoliter droplets simultaneously (Figure 1).

Using this 'Seq-on-a-Chip' on DMA methodology, hundreds of individual experiments involving cell culture, screening, phenotypic analysis, mRNA extraction from cells and subsequent conversion into cDNA for gene expression analysis by PCR and real-time PCR (qPCR) can all be accomplished simultaneously on a single chip. Until now, DMAs of two formats have been used, (i) 80-spot (5 × 16) arrays with circular spots of 3 mm diameter accommodating 5 pL total reaction volume [3 mm DMA], and (ii) 672-spot (14 × 48) arrays of square spots with 1 mm side length trapping droplets up to 200 nL volume [1 mm DMA] (Figure 2).

The present invention can be used in various technical fields, e.g. molecular genomics, personalized medicine, biotechnology etc. Moreover, cDNA can be barcoded in each nanoliter droplet with primers having unique molecular identifiers (UMIs), so that the cDNA from multiple droplets can be pooled and sequenced to reveal the global transcriptomic landscape of the cells in each droplet.

The present invention provides several advantageous properties.

First of all, the present invention avoids the high cost of conventional protocols for the isolation and manipulation of nucleic acid, this providing a reduction of the experimental budget: Specifically, the total reaction volume for 'Seq-on-a-Chip' is usually about 200 nanoliters. Hence, the protocol enables up to 100 times reduction of reagent consumption compared to standard protocols in tubes, assuming the usual reaction volume as 20 microliters (Table 1). This is important because the reagents used in molecular biology applications are expensive. For example, the cost of cDNA synthesis per reaction can be reduced by about EUR 14. In addition, there is evidence that small reaction volumes for gene expression studies lead to enhanced sensitivity, probably due to heightened molecular crowding.

**Table 1: Comparison of reagent volumes for cDNA synthesis in tube and on DMA with hydrophilic square spots of 3 mm and 1 mm DMA.**

| | **Reagent volume in PCR tube** (Usual reaction volume: 20 µL) | **Reagent volume per hydrophilic spot on DMA** | |
|---|---|---|---|
| | | 3 mm DMA (Total volume: 5 µL) | 1 mm DMA (Total volume: 200 nL) |
| ***Annealing primer to template RNA*** | | | |
| 10 mM dNTPs | 1 µL | 0.25 µL | 10 nL |
| 50 µM oligo dT primers or 50 ng/µL random hexamers | 1 µL | 0.25 µL | 10 nL |
| Nuclease free water | to 10 µL | to 2.25 µL | to 120 nL |

| ***Reverse transcription*** | | | |
|---|---|---|---|
| 5X reverse transcription buffer | 4 µL | 1 µL | 40 nL |
| 100 mM Dithiothreitol (DTT) | 1 µL | 0.25 µL | 10 nL |
| Ribonuclease inhibitor (40 U/µL) | 1 µL | 0.25 µL | 10 nL |
| Superscript IV reverse transcriptase (200 U/µL) | 1 µL | 0.25 µL | 10 nL |
| RNase H (2 U/µL) [optional] | 1 µL | 0.25 µL | 10 nL |

Further, the present invention can prevent the loss of the sample (*i.e.,* loss of the nucleic acid to be isolated) in large volumes due to non-specific absorption onto the surface of reaction vessels, which is a known problem in larger formats. Accordingly, the present invention allows of the efficient isolation of nucleic acids from low cell numbers. Since the 'wall-less' DMA is used for the protocol according to the present invention, the technical problem of non-specific adsorption of nucleic acids as observed in tubes and microtiter plates is solved. As a result, mRNA from as low as 10 mammalian cells can be successfully isolated, converted into cDNA and used for techniques like PCR and qPCR for subsequent analysis. Also, via introduction of UMI-barcoded primers, the 'Seq-on-a-Chip' protocol according to the present invention can be used to isolate sequencing-ready cDNA from cells cultured in nanoliter droplets.

Furthermore, commonly used protocols for the isolation and manipulation of nucleic acid are laborious, including multiple steps, and very time consuming. In contrast, the methodology according to the present invention offers a protocol with reduced number of steps. In addition, it reduces the hands-on time due to parallelization and multiplexing. In this context, state of the art methodologies are not multiplexed and use one sample at a time protocols. The methodology of the present invention, based on a DMA platform, enables parallel sample preparation in hundreds to thousands droplets per one slide/experiment.

Finally, state of the art methodologies offer no possibility to combine cell culture and/or screening with sample preparation (cell lysis, extraction, and manipulation of nucleic acid) on the same platform. Commonly cells are cultured in large volumes in multi-well plates, then sample preparation is performed in tubes, as single sample per tube. Transferring all these step onto the same platform, in nanoliter volumes *inter alia* poses the problem that microtiter plates are not compatible for the entire workflow of cell culture, screening, phenotypic assessment, mRNA Isolation and cDNA preparation for gene expression analysis. This is because the microtiter plates used for cell culture are not compatible for thermocycler machines needed for cDNA synthesis. Moreover, the usual protocol for mRNA isolation requires a number of centrifugation steps, which is also not compatible with the plates. The 'Seq-on-Chip' protocol according to the present invention, for the very first time, offers a unique possibility to combine cell screening, microscopic analysis, nucleic acid isolation and manipulation of the same in miniaturized volumes on DMA, thereby effectively increasing the experimental throughput and reducing the cost involving overconsumption of expensive reagents and plasticwares.

With the recent advancements in the field of molecular genomics, it is crucial to develop novel protocols enabling cell culture and high-throughput screening along with direct evaluation of cellular phenotype and gene expression dynamics. Through the novel 'Seq-on-Chip' protocol according to the present invention, the use of DMA as a simple, wall-free, high-throughput miniaturized readout platform for cell culture, incubation with various compounds/stimuli, imaging/viability assays and isolation of nucleic acids from cells for gene expression analysis and next generation sequencing has been demonstrated. Combining the entire set of experiments onto the same chip into nanoliter volumes allows performing hundreds of individual experiments simultaneously, with a great reduction in experimental budget.

The figures show:
Figure 1:
   Schematic workflow of gene expression analysis on DMA.
   1 = cell culture and screening in micro/nanoliter droplets on DMA, 2 = phenotypic analysis of cells by microscopy-based methods, 3 = cell lysis and isolation of mRNA using poly-T magnetic beads, 4 = mRNA to cDNA conversion within the same droplet, 5 = collection of cDNA for qualitative and quantitative gene expression analysis.
Figure 2:
   Schematic representation and detailed information about (A) 3 mm DMA and (B) 1 mm DMA. Scale bar = 5 mm.
Figure 3:
   Setup for building humidity chamber for DMA. Scale bar = 1 cm.
Figure 4:
   Droplet microarray with 3 mm DMA containing 5 µL nuclease free water (A) before and (B) after performing a cDNA synthesis program inside a thermocycler. Scale bar = 1 cm.
Figure 5:
   cDNA synthesis from total HeLa-CCL2 RNA on Droplet Microarray.
   (A) Schematic protocol for cDNA synthesis on 1 mm DMA. (B-E) Bright-field microscopic images of 5 µL (B-C) and 200 nL (D-E) droplets on hydrophilic spots of 3 mm and 1 mm DMA before (B and D) and after (C and E) the cDNA synthesis reaction, respectively. Scale bar= 500 µm. (F) Gel electrophoresis image of *GAPDH* PCR (product size =112 bp) performed with cDNA synthesized from 50 ng HeLa-CCL2 total RNA. [Lane number 1=1 kb DNA ladder, 2=No template (negative) control, 3=PCR product of cDNA synthesized in microtube (positive control), 4=PCR product of cDNA synthesized on 3 mm DMA in volume of 5 µL, 5=PCR product of cDNA synthesized on 50 hydrophilic spots of 1 mm DMA with volumes of 200 nL each.] (G) Gel electrophoresis image of *ACTB* PCR (product size =1045 bp) performed with cDNA synthesized from 50 ng HeLa-CCL2 total RNA. Sample sequence in gel lanes is same as in (F).
Figure 6:
   Detailed schematic outline of cell lysis, mRNA isolation and cDNA synthesis on 3 mm DMA according to 'Seq-on-a-Chip' methodology. RT= room temperature.
Figure 7:
   Bright-field microscopic images of HeLa-CCL2 cells cultured for 24 hours on 3 mm DMA, (A) before addition of lysis buffer, (B) 30 minutes after lysis buffer addition and (C) 1 hour after lysis buffer addition. Scale bar = 100 µM. (D) Bright-field microscopic images of 3 mm DMA at various steps of the entire protocol of cell lysis and cDNA synthesis via 'Seq-on-a-Chip' methodology. Scale bar = 500 µM.
Figure 8:
   Qualitative analysis and gene expression quantification for different number of cells and siRNA-mediated targeted gene knockdown on 3 mm DMA.
   (A-B) Gel electrophoresis image for *GAPDH* (product size = 112 bp) and *ACTB* (product size = 1045 bp) PCR respectively. [Lane number 1 = 1 kb DNA ladder, 2 = no template (negative) control, 3 = PCR product of cDNA synthesized in microtube from HeLa-CCL2 cells using standard protocol (positive control), 4 = PCR product of cDNA synthesized from HeLa-CCL2 cells on 3 mm DMA, via 'Seq-on-a-Chip' methodology]. (C-D) qPCR results of cDNA synthesized from HeLa-CCL2 cells our protocol on 3 mm DMA and PCR plate, respectively. Bar graphs from both C and D represent Ct values for 10, 100, 500 and 1000 cells, each with 5 replicates. Error bar represents mean±SEM. ****P*<0.001, ***P*<0.01 according to two-tailed unpaired *t*-test, calculated as comparison between consecutive groups. NS = not significant. Assessment of siGFP transfection to HeLa-GFP cells on 3 mm DMA: (E-G) Fluorescence microscopy images showing GFP expression in untransfected, scrambled siRNA-transfected and siGFPtransfected cells, respectively (after 48 hours of transfection). Scale bar = 100 µm. (H) Mean GFP fluorescence in HeLa-GFP cells post 48 hours of transfection. Data presented as mean±SEM from 3 individual experiments. ****P*<0.001, ***P*<0.01 according to two-tailed unpaired *t*-test. (I) qPCR for GFP transcript levels in cells post 48 hours of transfection on 3 mm DMA; cDNA synthesized via our protocol. Data normalized to *GAPDH* expression, and presented as mean±SEM, from 3 individual experiments. ****P*<0.001 as calculated by two-tailed unpaired t-test.
Figure 9:
   Bright-field microscopic images of HeLa-GFP cells cultured for 24 hours on 1 mm DMA, (A) before addition of lysis buffer and (B) 1 hour after lysis buffer addition. Scale bar = 100 µM. Fluorescence microscopic images of HeLa-GFP cells cultured for 24 hours on 1 mm DMA (C) before addition of lysis buffer and (D) 1 hour after lysis buffer addition. Scale bar = 100 µM. (E) Bright-field microscopic images of 1 mm DMA at various steps of mRNA isolation according to 'Cells to cDNA on Chip' protocol. Scale bar = 1 mm.
Figure 10:
   Representative fluorescent microscopy images of (A) HeLa-GFP cells cultured for 24 hours on 1 mm DMA (B) Cell counting for each hydrophilic spot on 1 mm DMA using analytical methods. Inset picture is for one hydrophilic spot with 1 mm side length. Scale bar = 1 mm.
Figure 11:
   Quantitative and qualitative gene expression analysis with different number of HeLa-GFP cells on 1 mm DMA.
   (A-B) Gel electrophoresis image for *GAPDH* (product size =112 bp) and *ACTB* (product size =1045 bp) PCR, respectively. [Lane number 1 = 1 kb DNA ladder, 2 = PCR product of cDNA synthesized from HeLa-GFP cells in microtube using standard protocol (positive control), 3 = PCR product of cDNA synthesized from HeLa-GFP cells on 1 mm DMA using our protocol, 4 = no template (negative) control]. (C) qPCR results of cDNA synthesized from HeLa-GFP cells using our protocol on 1 mm DMA. Synthesized cDNA was first pooled from 168 spots and then diluted so the content corresponding to a single droplet was used for qPCR reaction. Pink squares represent the average Ct value per hydrophilic spot from each cell number group. R = correlation coefficient. (D) Bright-field, fluorescent and overlaid microscope image of a single HeLa-GFP cell on 1 mm DMA. Scale bar = 100 µm. (E) qPCR amplification curves for 6 technical replicates of cDNA synthesized from single HeLa-GFP cells. NTC = no template control.
Figure 12:
   Real-time PCR analysis with single cells by 'Seq-on-a-Chip' methodology on 1 mm DMA. (A) Melting curve and (B) Melting peak analysis with 6 technical replicates for quantitative GFP expression in single HeLa-GFP cells. NTC = No template control.
Figure 13:
   Schematic outline for a representative experimental plan with 'Seq-on-a-Chip' protocol on DMA with cDNA barcoding in each spot. B1-B9 = barcoded primers, each with a unique molecular identifier.
Figure 14:
   Bioanalyzer report for doubled-stranded, amplified cDNA synthesized via 'Seq-on-a-Chip' protocol with cDNA barcoding.

The present invention will be further illustrated by the following example without being limited thereto.

### Example

The technical details characteristic to the 'Seq-on-a-Chip' protocol of the present invention, enabling cell lysis, mRNA isolation and cDNA synthesis along with barcoding on DMA are indicated below. The different cell lines used in screening experiments were continuously monitored at different steps of the protocol, and no cross-contamination between droplets was evident.

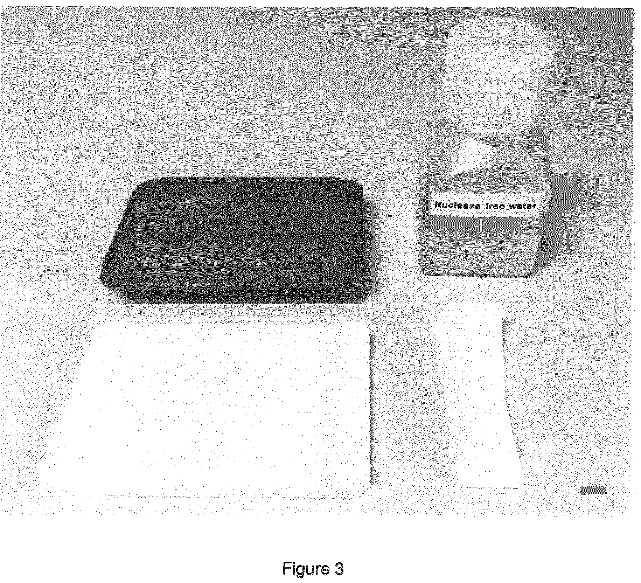

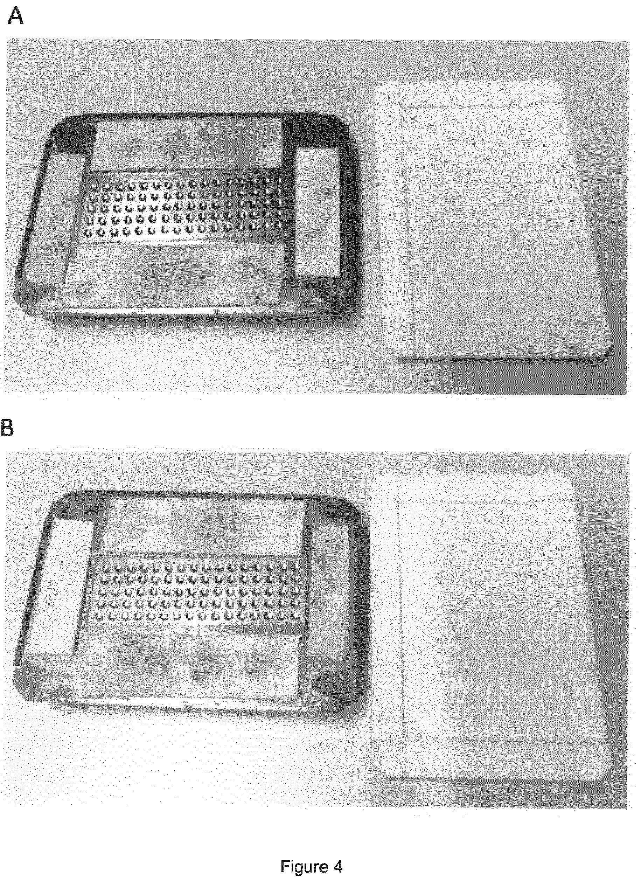

Few components were optimized towards building a 'humidity chamber' for DMA - (i) a metal *in situ* adaptor to fit inside the heating block of a thermocycler, (ii) a thermostable 3D printed lid to fix on top of the metal adaptor, and (iii) some paper strips wetted with appropriate volume of nuclease free water (Figure 3). This soformed humidity chamber was extensively tested for preventing evaporation of the droplets on DMA (Figure 4).

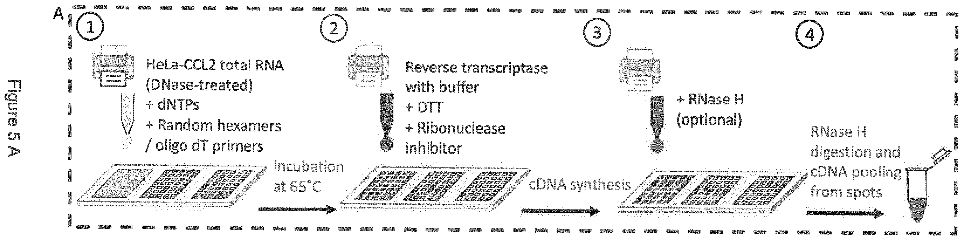

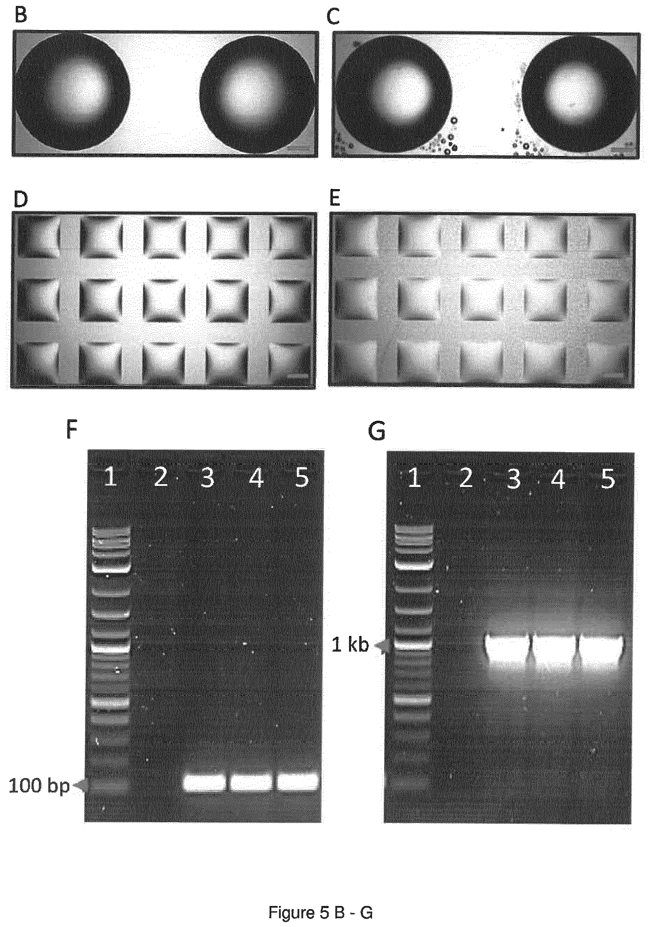

DNase-treated total HeLa-CCL2 RNA was added onto the hydrophilic spots on DMA, either by a nanoliter dispenser or manually. With a protocol in accordance with the present invention, the RNA could be successfully converted to cDNA on both DMA formats (Figure 5).

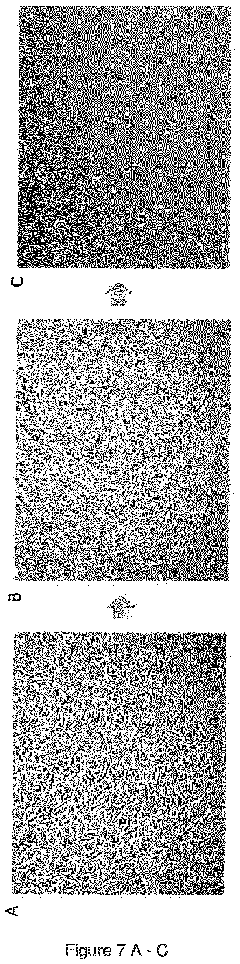

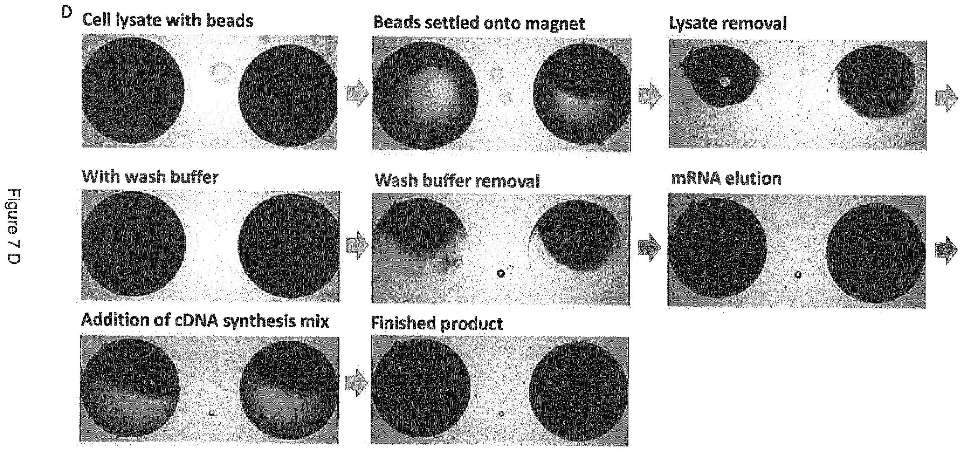

To begin with the 'Seq-on-a-Chip' protocol according to the present invention, cells on DMA were lysed with a lysis buffer, and mRNA was isolated using poly-T magnetic beads (Table 2). The resultant mRNA was used for cDNA synthesis on the same hydrophilic spots on DMA, followed by gene expression analysis via PCR and qPCR (Figure 6). No cross-contamination was evident while performing the protocol in 5 µl volume on 3mm DMA (Figure 7).

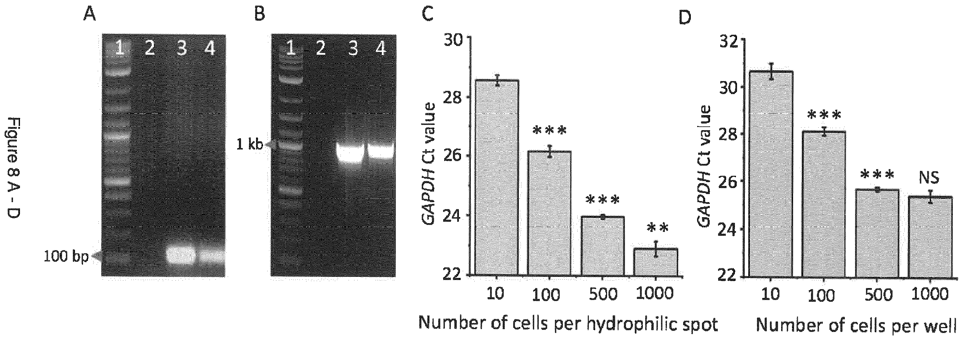

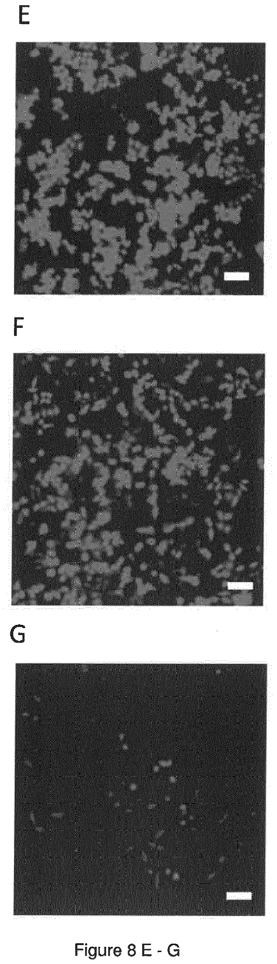

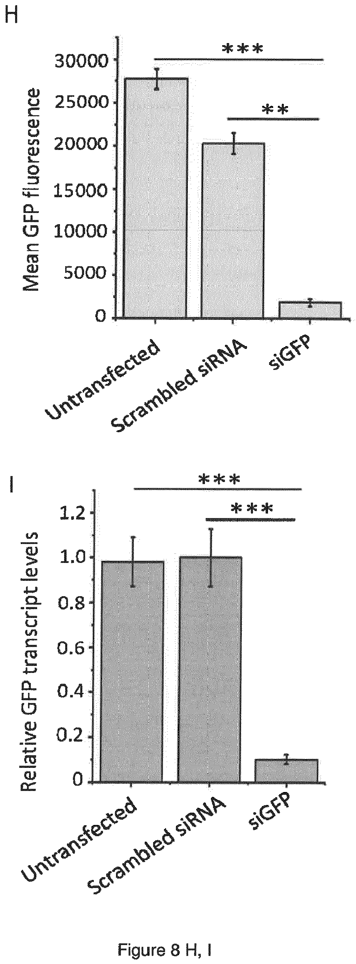

qPCR with differential number of cells on 3 mm DMA finally confirmed no cross-contamination between droplets during the entire protocol. The protocol according to the present invention is compatible with PCR plates, and demonstrates higher efficiency (Figure 8). As a suitable screening readout, cells on 3 mm DMA were transfected with small interfering RNAs (siRNA) against a specific target gene, and the entire workflow was performed. Results confirm the applicability of DMA to delineate the entire experimental course onto a single platform, with demonstrating about 90% knockdown of the target gene, with perfect congruence to the fluorescence microscopy-based readouts, (Figure 8).

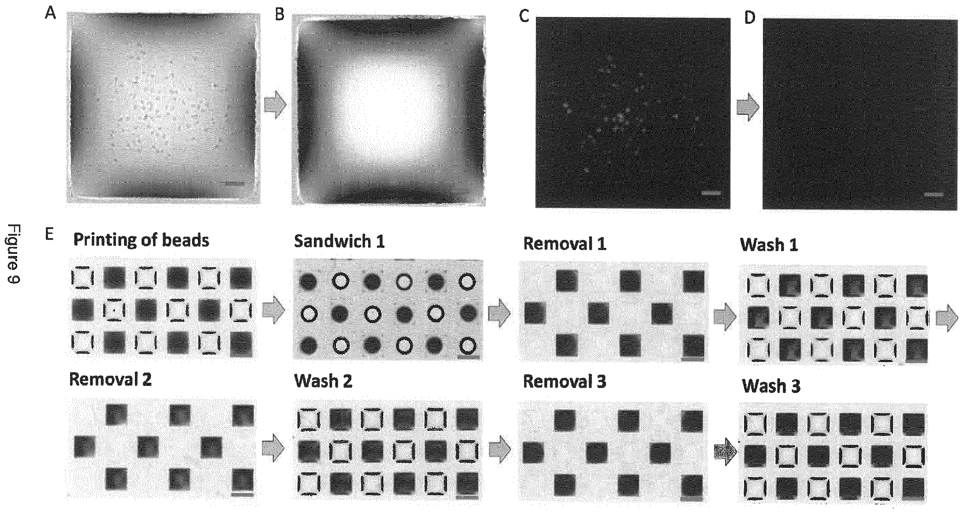

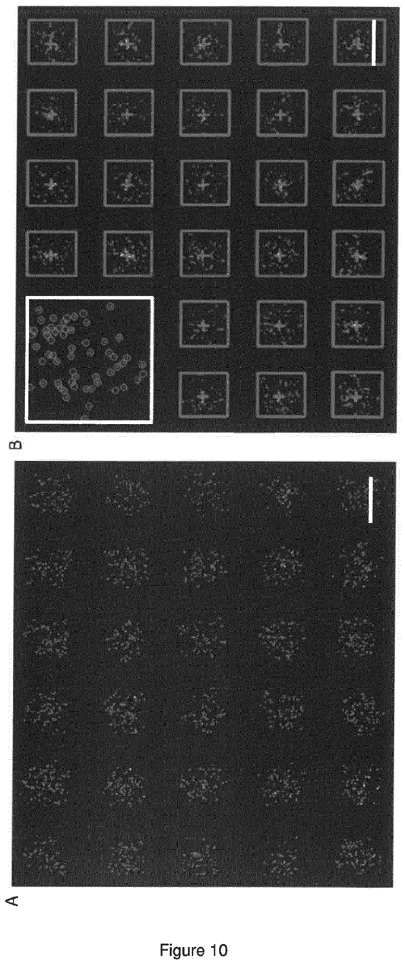

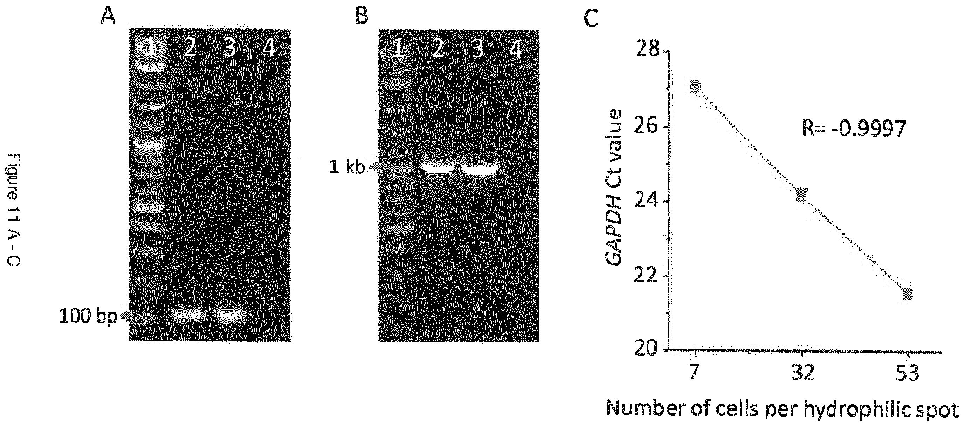

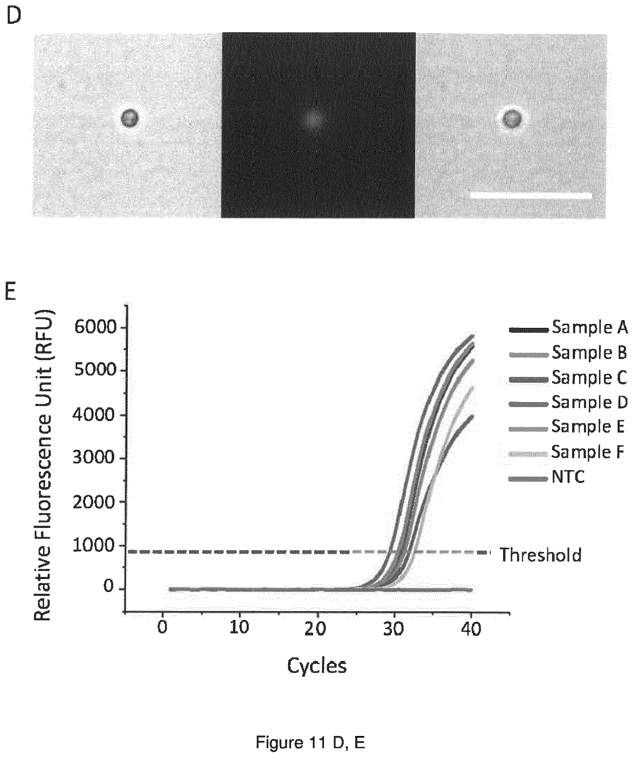

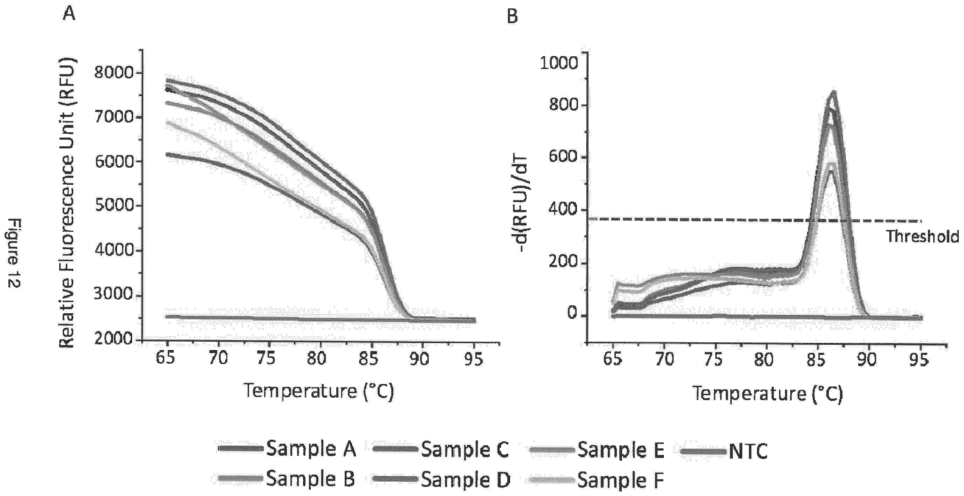

The entire 'Seq-on-a-Chip' protocol was further successfully miniaturized on 1 mm DMA, in a total reaction volume of 200 nL (Figure 9). For better stringency, cells in each droplet were imaged and counted using a in-house algorithm (Figure 10). The methodology was found appropriate to be performed within nanoliter volumes, as indicated by successful qualitative and quantitative gene expression analysis from different number of cells (Figure 11). Further, cDNA was synthesized from single cell per droplet, and quantified by qPCR (Figure 11). This finding further highlights the importance of having a wall-free nucleic acid isolation platform, so that genetic material from fewer number of cells can be extracted with better efficiency. Detailed analysis of qPCR curves for single-cell cDNA isolated by Seq-on-a-Chip on DMA yielded satisfactory results (Figure 12). Reagent volumes for mRNA isolation from cells on 1 mm and 3 mm DMA are mentioned in Table 2.

**Table 2: Sample/reagent volumes for cell seeding, lysis and mRNA isolation from cells on 3 mm and 1 mm DMA.**

| | **Reagent volume per hydrophilic spot** | |
|---|---|---|
| | 3 mm DMA (Total volume: 5 µL) | 1 mm DMA (Total volume: 200 nL) |
| ***Cell seeding*** | | |
| Number of cells | 1000 | 100 |
| Culture medium | 3 µL | 100 nL |

| ***mRNA isolation*** | | |
|---|---|---|
| Phosphate-buffered saline (×2) | - | 200 nL |
| Lysis buffer | 2 µL | 150 nL |
| Poly-T magnetic beads in hybridization buffer | 3 µL | 100 nL |
| Wash buffer (×3) | 6 µL | 200 nL |
| Nuclease free water for mRNA elution | 2.5 µL | 100 nL |

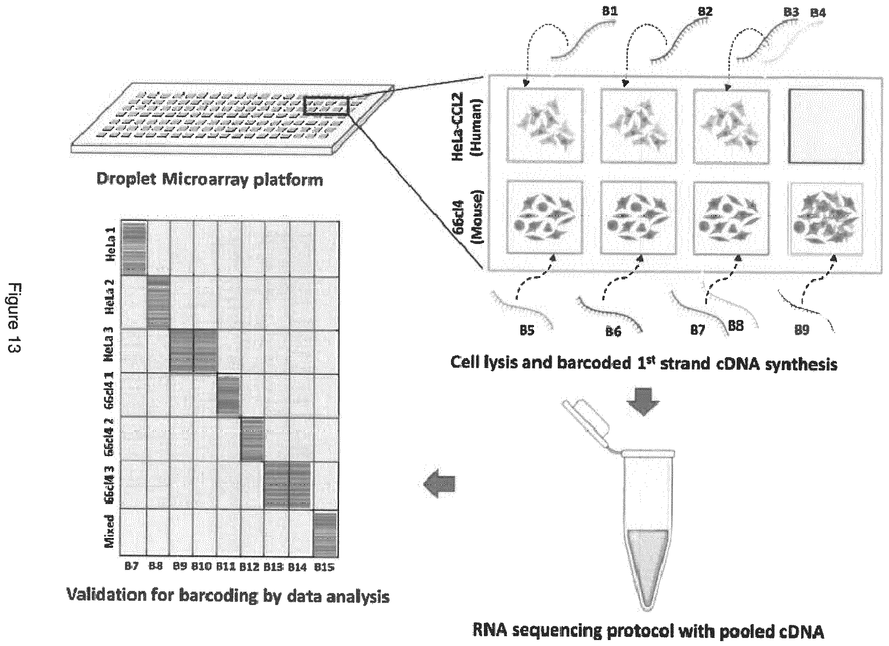

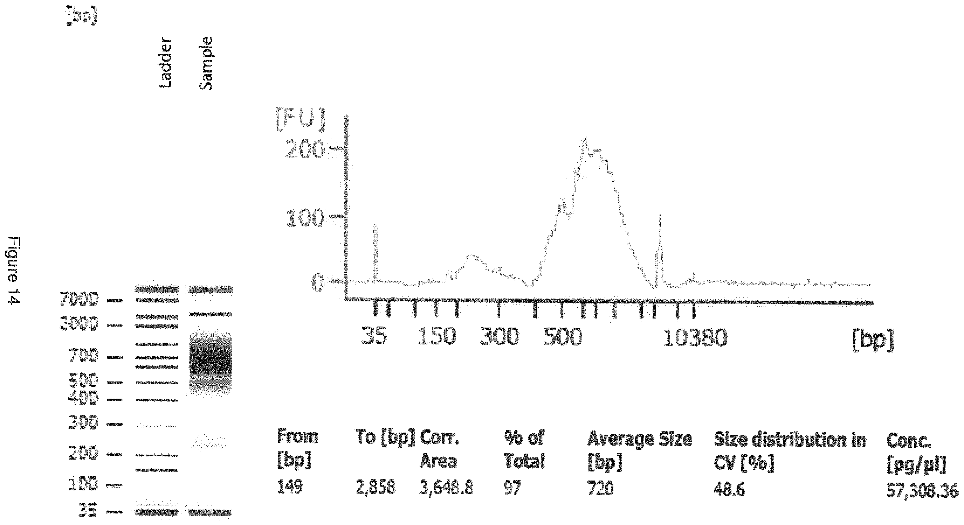

As one step further towards evaluation of the global transcriptomic landscape of cells post screening on DMA, UMI-barcoded primers have been introduced to each nanoliter droplet during cDNA synthesis on DMA. Then, the cDNA from each droplet could be pooled, and the transcriptome of each sample analyzed via next generation sequencing (Figure 13). Full-length double-stranded cDNA was generated on DMA, amplified by PCR, and quality-checked by bioanalyzer in preparation for sequencing studies (Figure 14).

## Claims

1. A method for the direct and parallel isolation of nucleic acid from cells in a multitude of nano- or microdroplets, comprising the steps of:
(a) providing a patterned substrate comprising a solid support coated with or carrying a layer or film, said layer or film comprising
(i) a multitude of hydrophilic areas having a desired shape and size, surrounded by
(ii) hydrophobic areas separating the hydrophilic areas into a desired spatial pattern;
(b) applying an aqueous fluid comprising cells to the multitude of hydrophilic areas, thereby forming an array of a multitude of separated aqueous fluid nano- or microdroplets, wherein the separated aqueous fluid nano- or microdroplets comprise cells; and
(c) isolating nucleic acid from the cells in the multitude of separated aqueous fluid nano- or microdroplets on the patterned substrate in parallel.

2. The method of claim 1, further comprising the step of (b-2) culturing the cells in the multitude of separated aqueous fluid nano- or microdroplets on the patterned substrate after step (b) and prior to step (c).

3. The method of claim 1 or claim 2, further comprising the step of (b-3) analyzing the cells in the multitude of separated aqueous fluid nano- or microdroplets on the patterned substrate by way of microscopy after step (b) and prior to step (c).

4. The method of any one of claims 1 to 3, wherein the nucleic acid is DNA or RNA.

5. The method of any one of claims 1 to 4, wherein the nucleic acid is mRNA.

6. The method of claim 5, further comprising the step of (d) synthesizing cDNA in the multitude of separated aqueous fluid nano- or microdroplets on the patterned substrate in parallel from the mRNA isolated in step (c).

7. The method of any one of claims 1 to 6, said method further comprising one or more steps, selected from the group consisting of introducing barcodes to the nucleic acid, amplifying the nucleic acid, performing PCR (polymerase chain reaction) with the nucleic acid, performing qPCR (quantitative PCR) with the nucleic acid, and sequencing the nucleic acid, in the multitude of separated aqueous fluid nano- or microdroplets on the patterned substrate in parallel using the nucleic acid isolated in step (c).

8. The method of any one of claims 1 to 7, wherein the separated aqueous fluid nano- or microdroplets have a volume of about 5 nL to about 10 µL.

9. The method of any one of claims 1 to 8, wherein the aqueous fluid is selected from the group consisting of water, aqueous solutions, and aqueous media.

10. The method of any one of claims 1 to 9, wherein the solid support is an optically transparent plastic slide or a glass slide.

11. The method of any one of claims 1 to 10, wherein the layer or film coated onto or carried by the solid support of the patterned substrate is selected from the group consisting of (i) a micro-to-nanorough inorganic film coated on the patterned substrate, (ii) a porous polymer layer or film coated on the patterned substrate, (iii) a micro-to-nanorough layer, and (iv) a smooth coating with omniphilic-omniphobic patterning.

12. The method of claim 11, wherein the layer or film is a micro-to-nanorough, inorganic, nanoparticle-based film coated on the patterned substrate.

13. The method of any one of claims 1 to 12, wherein the layer or film coated onto the solid support of the patterned substrate has a thickness of 45 µm or less.

14. The method of any one of claims 1 to 13, wherein applying an aqueous fluid comprising cells to the multitude of hydrophilic areas in step (b) is effected by discontinuous wetting, or on a spot-by-spot basis using liquid dispensers.

15. The method of any one of claims 1 to 14, wherein the number of cells per individual nano- or microdroplet is 1000 cells or less, 100 cells or less, 10 cells or less, or one single cell.
